# DEMANDE DE BREVET EUROPEEN

(11) **EP 2 359 804 A2**
(43) Date de publication de la demande: **24.08.2011**
(21) Numéro de dépôt: 11152472.4
(22) Date de dépôt: 28.01.2011
(51) Int. Cl.: A61K 8/41, A61K 8/55, A61Q 5/06, A61Q 5/10

(54) **Composition comprenant un colorant d'oxydation et un derive d'hydroxyde d'ammonium ou de phosphonuim, coloration de fibres keratiniques et dispositif**

(30) Priorité: 28.01.2010 FR 1050566; 28.01.2010 FR 1050568; 28.01.2010 FR 1050564
(71) Demandeur: L'Oréal, 75008 Paris (FR)
(72) Inventeur: Lalleman, Boris, 75004, Paris (FR); Lagrange, Alain, 77700, Coupvray (FR)
(74) Mandataire: Wattremez, Catherine

(57) **Abrégé**

La présente invention a pour objet des compositions comprenant dans un milieu cosmétiquement acceptable, un ou plusieurs colorants d'oxydation et un ou plusieurs composés choisis parmi les composés de formules (I) et (I') suivantes
(I) hydroxyde d'ammonium quaternaire de formule (I) : 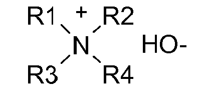 (I') hydroxyde de phosphonium quaternaire de formule (I') : 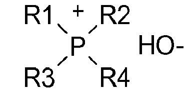 dans laquelle R₁, R₂, R₃, R₄ identiques ou différents représentent des radicaux alkyles en C₁-C₁₀ éventuellement substitués par un ou plusieurs atomes d'halogène, radicaux hydroxyle, alcoxy en C₁-C₄, carboxylique dans le cas de (I') ; des radicaux alkyl(C₁-C₂)aryle (C₆) dans le cas de (I).

Elle a également pour objet un procédé de coloration de fibres kératiniques humaines mettant en jeu une telle composition.

Elle a aussi pour objet un kit comprenant au moins une composition selon l'invention ne contenant pas d'agent oxydant et une composition oxydante comprenant, dans un milieu cosmétiquement acceptable, un ou plusieurs agents oxydants.

## Description

La présente invention a pour objet des compositions de coloration de fibres kératiniques humaines, comprenant dans un milieu cosmétiquement acceptable, au moins un colorant d'oxydation et au moins un dérivé d'hydroxyde d'ammonium ou de phosphonium particulier.

Elle a également pour objet un procédé de coloration de fibres kératiniques humaines mettant en jeu une telle composition, ainsi qu'un dispositif pour sa mise en oeuvre.

On connaît deux grands modes de coloration des fibres kératiniques humaines, et en particulier des cheveux.

Le premier, appelé coloration d'oxydation ou permanente, consiste à mettre en oeuvre un ou plusieurs colorants d'oxydation, plus particulièrement une ou plusieurs bases d'oxydation éventuellement associées à un ou plusieurs coupleurs.

Habituellement, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder, par un processus de condensation oxydative, à des espèces colorées qui restent piégées à l'intérieur de la fibre.

Bien souvent, on fait varier les nuances obtenues avec ces bases d'oxydation en les associant à un ou plusieurs coupleurs, ces derniers étant choisis notamment parmi les métadiamines aromatiques, les méta-aminophénols, les métadiphénols et certains composés hétérocycliques, tels que des composés indoliques.

La variété des molécules mises en jeu au niveau des bases d'oxydation et des coupleurs permet l'obtention d'une riche palette de couleurs.

Ce type de coloration permet également l'obtention de colorations permanentes, mais l'utilisation d'agents oxydants, très souvent dans des conditions de pH alcalin, peut entraîner une dégradation des fibres kératiniques.

On est également confronté à des difficultés provenant de la nature de l'agent alcalin le plus couramment utilisé, et qui est l'ammoniaque. D'un côté, l'emploi de ce composé est particulièrement avantageux car il permet non seulement d'ajuster le pH pour permettre l'activation de l'agent oxydant, mais provoque également un gonflement de la fibre, avec une ouverture des écailles, qui favorise la pénétration de l'agent oxydant et augmente l'efficacité de la réaction. Mais d'un autre côté, cet agent alcalinisant est très volatil et cela nécessite de le mettre en oeuvre avec des teneurs relativement importantes pour en compenser les pertes, avec pour conséquence de causer des désagréments du fait de l'odeur caractéristique de ce composé.

Le deuxième mode de coloration, appelé coloration directe ou semi-permanente, comprend l'application de colorants directs qui sont des molécules ayant une affinité pour les fibres et colorantes même en l'absence d'agent oxydant ajouté dans les compositions les contenant. Etant donnée la nature des molécules employées, celles-ci restent plutôt en surface de la fibre et pénètrent relativement peu à l'intérieur de la fibre, comparées aux petites molécules de colorants d'oxydation.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. Les espèces chimiques mises en oeuvre peuvent être non ioniques, anioniques (colorants acides) ou cationiques (colorants basiques). Les colorants directs peuvent également être des colorants naturels.

Ces compositions contenant un ou plusieurs colorants directs sont appliquées sur les fibres kératiniques pendant un temps nécessaire à l'obtention de la coloration désirée, puis rincées.

Cependant, les colorations qui en résultent sont des colorations particulièrement chromatiques mais temporaires ou semi-permanentes car leur désorption de la surface et/ou du coeur de la fibre sont responsables de leur faible puissance tinctoriale et de leur mauvaise tenue aux lavages.

Le but de la présente invention est de fournir de nouvelles compositions pour la teinture des cheveux humains qui permettent des teintures puissantes, résistantes, peu sélectives et pouvant également donner des nuances variées.

Par ailleurs, l'invention permet également de proposer un agent de remplacement en tout ou partie, de l'ammoniaque, sans dégrader les propriétés tinctoriales de la composition.

Ainsi, la présente invention a pour objet une composition tinctoriale comprenant, comprenant dans un milieu cosmétiquement acceptable, un ou plusieurs colorants capillaires, et un ou plusieurs composés choisis parmi les composés de formules (1) et (1') suivantes
(1) hydroxyde d'ammonium quaternaire de formule (1) suivante : 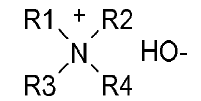 dans laquelle R₁, R₂, R₃, R₄, identiques ou différents représentent des radicaux alkyles en C₁-C₂₀, plus particulièrement en C₁-C₁₀, éventuellement substitués par un groupement choisi parmi les atomes d'halogène, les groupements hydroxyle, alcoxy en C₁-C₄; des radicaux alkyl(C₁-C₂)aryle (C₆) ;
(1') hydroxyde de phosphonium quaternaire de formule (l') suivante : 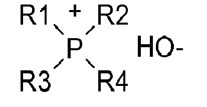
dans laquelle R₁, R₂, R₃, R₄ identiques ou différents représentent des radicaux alkyles en C₁-C₁₀ éventuellement substitués par un ou plusieurs atomes d'halogène, en particulier le chlore, le brome ou l'iode, un ou plusieurs radicaux hydroxyle, alcoxy en C₁-C₄, carboxylique.

Elle a également pour objet un procédé de coloration de fibres kératiniques humaines et en particulier les cheveux, consistant à appliquer sur lesdites fibres, la composition selon l'invention.

Elle a aussi pour autre objet un dispositif multi-compartiments ou kit comprenant au moins une composition selon l'invention, dépourvue d'agent oxydant, et une composition oxydante comprenant, dans un milieu cosmétiquement acceptable, un ou plusieurs agents oxydants.

D'autres caractéristiques et avantages de l'invention apparaîtront plus clairement à la lecture de la description et des exemples.

Il est à noter que dans ce qui va suivre, et à moins d'une autre indication, les bornes d'un domaine de valeurs sont comprises dans ce domaine.

Comme indiqué précédemment, la composition tinctoriale selon l'invention comprend un ou plusieurs colorants capillaires.

Par colorant capillaire on entend au sens de la présente invention les colorants directs synthétiques, les colorants naturels, ainsi que les précurseurs de colorants d'oxydation, ou leurs mélanges.

En particulier, ces colorants capillaires peuvent être non ioniques ou ioniques, en particulier cationiques ou anioniques.

Par colorants naturels, on entend tout colorant ou précurseur de colorant ayant une occurrence naturelle et produit soit par extraction (et éventuellement purification) depuis une matrice végétale, soit par synthèse chimique.

A contrario on entend par colorants synthétiques on entend tout colorant n'ayant pas une occurrence naturelle.

Dans un premier mode de réalisation, la composition selon l'invention comprend un ou plusieurs colorants d'oxydation, choisis parmi les bases d'oxydation et les coupleurs, et leurs mélanges.

De préférence les colorants d'oxydation comprennent au moins une base d'oxydation.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-βhydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de dérivés pyrazoliques on peut également citer les diamino N,N-dihydropyrazolopyrazolones et notamment celles décrites dans la demande FR 2886136 telles que les composés suivants et leurs sels d'addition : 2,3-Diamino-6,7-dihydro-1H,5H-pyrazolo[1,2-a]pyrazol-1-one, 2-Amino-3-éthylamino-6,7-dihydro-1H,5H-pyrazolo[1,2-a]pyrazol-1-one, 2-Amino-3-isopropylamino-6,7-dihydro-1H,5H-pyrazolo[1,2-a]pyrazol-1-one, 2-Amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-Amino-1,2-diethyl-5-(pyrrolidin-1-yl)-1,2-dihydro-pyrazol-3-one, 4-Amino-5-(3-dimethylamino-pyrrolidin-1-yl)-1,2-diethyl-1,2-dihydro-pyrazol-3-one, 2,3-diamino-6-hydroxy-6,7-dihydro-1H,5H-pyrazolo[1,2-a]pyrazol-1-one.

A titre de bases hétérocycliques on utilisera préférentiellement le 4,5-diamino 1-(β-hydroxyéthyl) pyrazole et/ou la 2,3-diamino-6,7-dihydro-1H,5H-pyrazolo[1,2,A]pyrazol-1-one et leurs sels d'addition.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-fl-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(f1-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation, quand elles sont présentes dans la composition, représentent de préférence de 0,0001 à 10 % en poids par rapport au poids de la composition, et de préférence de 0,005 à 5 % en poids par rapport au poids de la composition.

Le ou les coupleurs, s'ils sont présents, représentent de préférence de 0,0001 à 10 % en poids par rapport au poids de la composition, et de préférence de 0,005 à 5 % en poids par rapport au poids de la composition.

Dans un deuxième mode de réalisation, la composition comprend un ou plusieurs colorants directs synthétiques ou naturels.

A titre d'exemples de colorants directs synthétiques convenables, on peut citer les colorants directs azoïques ; méthiniques ; carbonyles ; aziniques ; nitrés (hétéro)aryle ; tri-(hétéro)aryle méthanes ; seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine vraie (comprenant un ou plusieurs enchaînements -C=C- précités) ; azométhine (comprenant au moins un ou plusieurs enchaînements -C=N-) avec par exemple les azacabocyanines et leurs isomères, les diazacarbocyanines et leurs isomères, les tétraazacarbocyanines ; mono-et di- arylméthane ; indoamines (ou diphénylamines) ; indophénols ; indoanilines.

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des aziniques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; méthiniques ; azométhiniques avec plus particulièrement les diazacarbocyanines et leurs isomères et les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines et porphyrines ; seuls ou en mélanges.

De préférence, les colorants directs sont choisis parmi les colorants nitrés de la série benzénique ; azoïques ; azométhines avec les diazacarbocyanines et leurs isomères, les tétraazacarbocyanines (tétraazapentaméthines) ; les colorants directs anthraquinoniques ; les colorants directs triarylméthaniques ; seuls ou en mélanges.

De manière encore plus préférée, ces colorants directs sont choisis parmi les colorants nitrés de la série benzénique ; les colorants directs azoïques ; azométhines avec les diazacarbocyanines et leurs isomères, les tétraazacarbocyanines (tétraazapentaméthines) ; seuls ou en mélange.

Parmi les colorants directs nitrés benzéniques utilisables selon l'invention, on peut citer de manière non limitative les composés suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylam ino)-2-nitrobenzène
- 1-β-hydroxyéthyiamino-2-nitro-4-bis-(β-hydroxyéthyiamino)-benzène
- 1-β-hydroxyéthyiamino-2-nitro-4-aminobenzène
- 1-β-hydroxyéthyiamino-2-nitro-4-(éthyl)(β-hydroxyéthyi)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyioxy-3-méthyiamino-4-nitrobenzène
- 1-βy-dihydroxypropyloxy-3-méthyiamino-4-nitrobenzène
- 1-β-hydroxyéthylamino-4-β,y-dihydroxypropyloxy-2-nitrobenzène
- 1-βy-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines utilisables selon l'invention on peut citer les colorants cationiques décrits dans les demandes de brevets WO 95/15144, WO 95/01772 et EP 714954 ; FR 2189006, FR 2285851, FR 2140205, EP 1378544, EP 1674073.

Ainsi, on peut tout notamment citer les colorants directs cationiques correspondants aux formules suivantes : 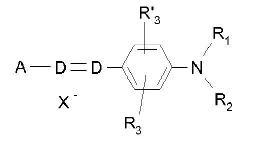 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X - représente un anion de préférence choisi parmi le chlorure, le méthyl sulfate et l'acétate,
A représente un groupement choisi par les structures suivantes : 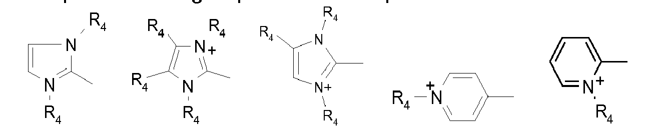 dans lesquelles R₄ représente un radical alkyle en C₁-C₄ pouvant être substitué par un radical hydroxyle ; 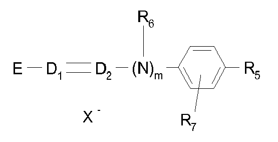 dans lesquelles :
   R₅ représente un atome d'hydrogène, un radical alcoxy en C₁-C₄, un atome d'halogène tel que le brome, le chlore, l'iode ou le fluor,
   R₆ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₇ représente un atome d'hydrogène ou d'halogène tel que le brome, le chlore, l'iode ou le fluor,
   D₁ et D₂, identiques ou différents, représentent un atome d'azote ou le groupement -CH, m = 0 ou 1,
   X - représente un anion cosmétiquement acceptable et de préférence choisi parmi le chlorure, le méthyl sulfate et l'acétate,
   E représente un groupement choisi par les structures suivantes : 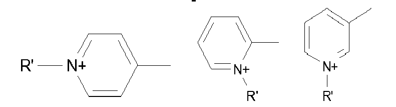 dans lesquelles R' représente un radical alkyle en C₁-C₄ ;
      lorsque m = 0 et que D₁ représente un atome d'azote, alors E peut également désigner un groupement de structure suivante : 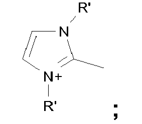 dans laquelle R' représente un radical alkyle en C₁-C_{4.}

Parmi les composés précités, on utilise tout particulièrement les composés suivants :

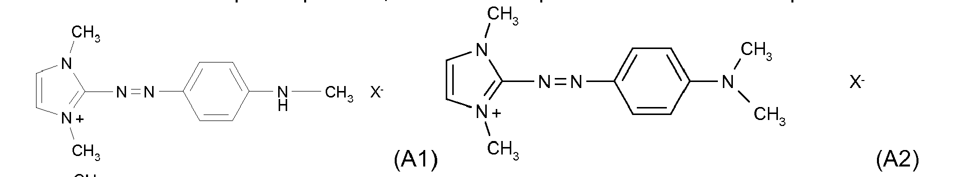

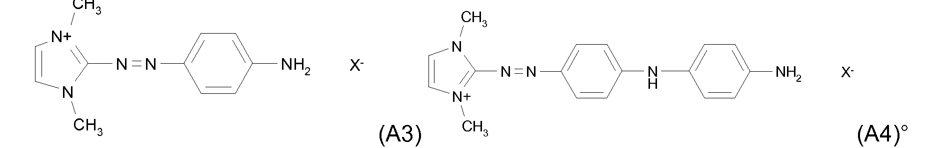

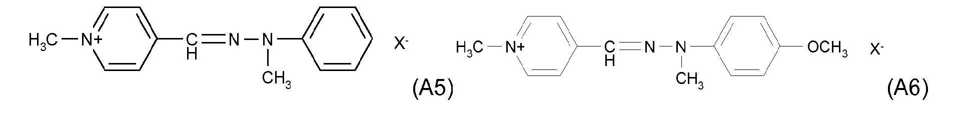

Parmi les colorants de type tétraazapentaméthiniques utilisables selon l'invention, on peut citer les composés suivants figurant dans le tableau ci-dessous :

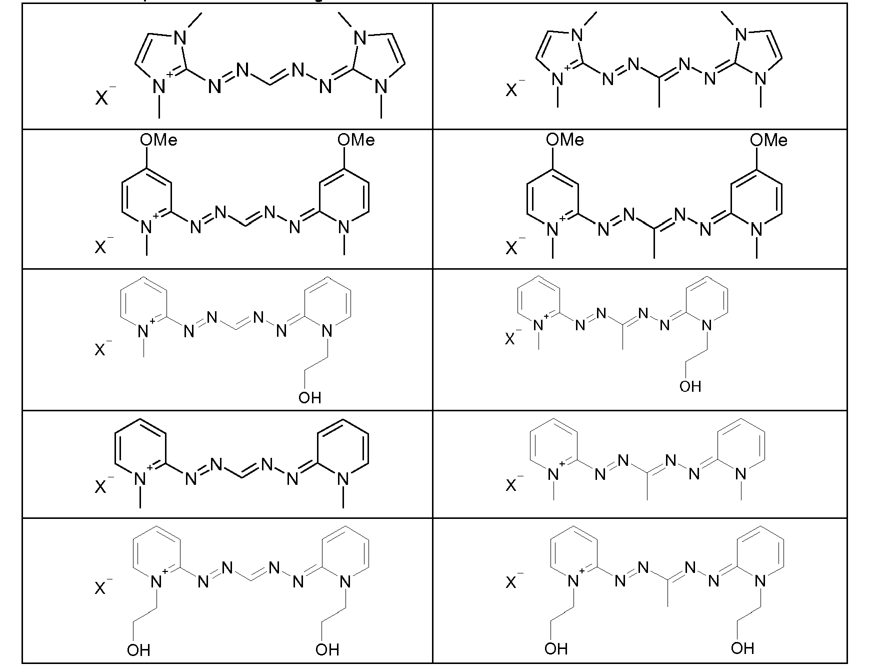

X représente un anion ou un mélange d'anions cosmétiquement acceptables, de préférence choisi parmi le chlorure, l'iodure, le méthyl sulfate, l'éthyl sulfate, l'acétate.

Comme autres colorants utilisables selon l'invention on peut également citer parmi les colorants directs azoïques les colorants suivants, décrits dans le COLOUR INDEX INTERNATIONAL 3e édition :
- Disperse Red 17
- Disperse Red 13
- Basic Red 22
- Basic Red 76
- Basic Yellow 57
- Basic Brown 16
- Basic Brown 17
- Disperse Green 9
- Disperse Black 9.
- Solvent Black 3
- Disperse Blue 148
- Disperse Violet 63
- Solvent Orange 7

On peut aussi citer le 1-(4'-aminodiphénylazo)-2-méthyl-4bis-(β-hydroxyéthyl) aminobenzène (Nom INCI : HC Yellow 7).

Parmi les colorants directs quinoniques on peut citer les colorants suivants :
- Disperse Red 15
- Solvent Violet 13
- Solvent Blue 14
- Disperse Violet 1
- Disperse Violet 4
- Disperse Blue 1
- Disperse Violet 8
- Disperse Blue 3
- Disperse Red 11
- Disperse Blue 7
- Disperse Blue 14
- Basic Blue 22
- Disperse Violet 15
- Disperse Blue 377
- Disperse Blue 60
- Basic Blue 99
   ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(βy-dihydroxypropylamino)-anthraquinone.

On peut aussi citer la coumarine Disperse Yellow 82.

Parmi les colorants aziniques on peut citer les composés suivants :
- Basic Blue 17
- Basic Red 2
- Solvent Orange 15.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthylamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

De préférence, les colorants directs cationiques sont choisis parmi les colorants directs de types azoïques ; méthines ; azométhines avec les diazacarbocyanines et leurs isomères, les tétraazacarbocyanines (tétraazapentaméthines) ; anthraquinoniques ; seuls ou en mélange.

Pour les colorants non ioniques on préfère tout particulièrement les composés présentant un LogP supérieur ou égal à 2.

En ce qui concerne les colorants directs synthétiques de LogP supérieur ou égal à 2, il est rappelé que la valeur du logP représente de façon classique le coefficient de partage du colorant entre l'octanol et l'eau. Le logP peut être calculé selon la méthode décrite dans l'article de Meylan et Howard « Atom l Fragment contribution method for estimating octanol-water partition coefficient », J. Pharm. Sci. 84 :83-92, 1995. Cette valeur peut aussi être calculée à partir de nombreux logiciels disponibles sur le marché qui détermine le logP en fonction de la structure d'une molécule. A titre d'exemple, on peut citer le logiciel Epiwin de l'agence de l'environnement des Etats-Unis.

En particulier, les colorants convenant à la mise en oeuvre de l'invention sont choisis parmi les composés suivants, seuls ou en mélange :

| **Colorant** | **Structure chimique** | **logP** |
|---|---|---|
| Disperse Red 17 | 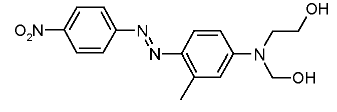 | 3.69 |
| Disperse Violet 1 | 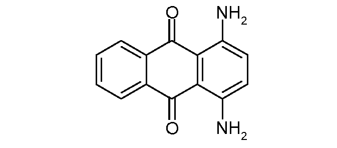 | 3.0 |
| HC Yellow 7 | 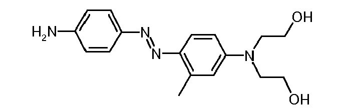 | 2.38 |
| Disperse Blue 377 | 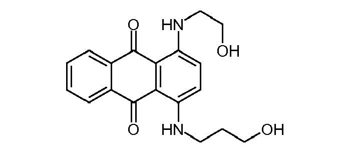 | 3.21 |
| Disperse Red 13 | 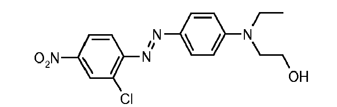 | 5.22 |
| Disperse Green 9 | 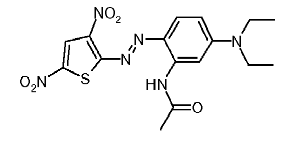 | 4.23 |
| Solvent Black 3 | 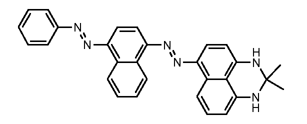 | 7.50 |
| Disperse Blue 148 | 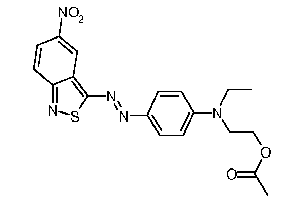 | 4.81 |
| Disperse Violet 63 | 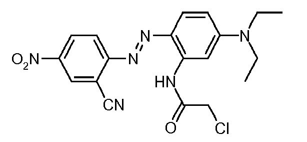 | 5.30 |
| Disperse Blue 60 | 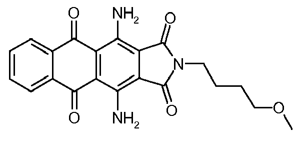 | 3.38 |
| Disperse Blue 14 | 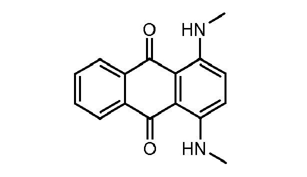 | 4.25 |
| Solvent Orange 15 | 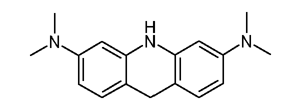 | 3.90 |
| Solvent Orange 7 | 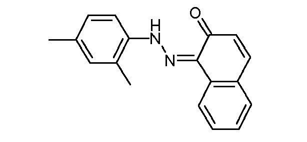 | 4.40 |
| Solvent Blue 14 | 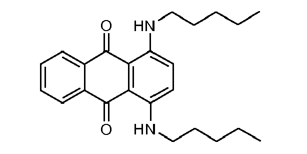 | 8.18 |
| Disperse Yellow 82 | 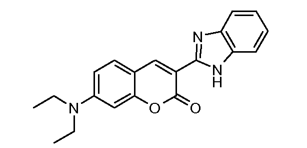 | 3.68 |

Encore plus préférentiellement, les colorants directs de l'invention sont choisis parmi les colorants cationiques de types azoïques ; méthines ; azométhines avec les diazacarbocyanines et leurs isomères, les tétraazacarbocyanines (tétraazapentaméthines) ; anthraquinoniques ; seuls ou en mélange, et en particulier les colorants (A1 ) à (A6) cités précédemment, ainsi que les colorants non ioniques de logP supérieur ou égal à 2.

Parmi les colorants directs anioniques on peut en particulier citer ceux décrits dans le COLOUR INDEX INTERNATIONAL 3e édition sous l'appellation ACID et en particulier :
- Disperse Red 17
- Acid Yellow 9
- Acid Black 1
- Acid Yellow 36
- Acid Orange 7
- Acid Red 33
- Acid Red 35
- Acid Yellow 23
- Acid Orange 24
- Acid Violet 43
- Acid Blue 62
- Acid blue 9
- Acid Violet 49
- Acid Blue 7.

Le ou les colorants directs synthétiques, quand ils sont présents, représentent de préférence de 0,0001 à 10 % en poids par rapport au poids de la composition, et de préférence de 0,005 à 5 % en poids par rapport à la même référence.

La composition selon l'invention peut comprendre un ou plusieurs colorants naturels.

Le ou les colorants naturels convenables en particulier à la mise en oeuvre de l'invention sont choisis de préférence parmi la lawsone, la juglone, l'alizarine, la purpurine, l'acide carminique, l'acide kermésique, l'acide laccaïque, la purpurogalline, l'anthragallol, la protocatéchaldéhyde, l'indigo, l'isatine, la curcumine, la spinusoline, les chlorophylles, les chlorophyllines, les orcéines, l'hématéine, l'hématoxyline, la braziline, la braziléine, les colorants du carthame (comme par exemple la carthamine), les flavonoïdes (avec par exemple la morine, l'apigénidine, le santal), les anthocyanes (du type de l'apigéninidine), les caroténoïdes, les tanins, les colorants de sorgho et le carmin de cochenille, ou leurs mélanges.

On peut également utiliser les extraits ou décoctions contenant ces colorants naturels et notamment les extraits à base de henné.

De préférence, le ou les colorants naturels sont choisis parmi la lawsone, la juglone, l'alizarine, la purpurine, l'acide carminique, l'acide kermésique, l'acide laccaïque, la purpurogalline, le protocatéchaldéhyde, l'indigo, l'isatine, la curcumine, la spinulosine, l'apigénidine, la chlorophylline, les extraits de sorgho, les orcéines, le carmin de cochenille, l'hématéine, l'hématoxyline, la braziline et braziléine, et leurs mélanges.

Ces colorants peuvent éventuellement être utilisés en présence de mordants (ex sels de zinc, de manganèse, d'aluminium, de fer, ...)

Les colorants naturels, quand ils sont présents, représentent de préférence de 0,001 à 10% en poids, de préférence de 0,01 à 8 % en poids, mieux de 0,1 à 5% en poids, par rapport au poids de la composition.

Comme indiqué auparavant, la composition selon l'invention comprend un ou plusieurs composés de formule (l) ou de formule (l')

Les hydroxydes d'ammonium quaternaires correspondent à la formule (l) suivante : 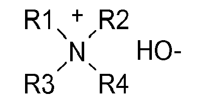 dans laquelle R₁, R₂, R₃, R₄, identiques ou différents représentent des radicaux alkyles en C₁-C₂₀, plus particulièrement en C₁-C₁₀, éventuellement substitués par un groupement choisi parmi les atomes d'halogène, les groupements hydroxyle, alcoxy en C₁-C₄ ; des radicaux alkyl(C₁-C₂)aryle (C₆).

Avantageusement, le composé de formule (l) est choisi parmi l'hydroxyde de méthyltriéthanol ammonium, l'hydroxyde de tétraéthyl ammonium, l'hydroxyde de benzyltriméthyl ammonium, l'hydroxyde de tétraméthyl ammonium, l'hydroxyde de tétrabutyl ammonium, l'hydroxyde de tétrapropyl ammonium, l'hydroxyde de méthyl triéthanol ammonium, l'hydroxyde de benzyl diméthyl éthanol ammonium, l'hydroxyde de benzyl triéthyl ammonium, l'hydroxyde de benzyl tributyl ammonium, l'hydroxyde d'octadécyl triméthyl ammonium, l'hydroxyde de tétrahexyl ammonium, l'hydroxyde de tétraoctyl ammonium, ou leurs mélanges.

Selon une variante préférée de l'invention, le composé de formule (l) est tel que les radicaux R₁, R₂, R₃, R₄, identiques ou différents représentent des radicaux alkyles en C₂-C₆, éventuellement substitués par un groupement choisi parmi les groupements hydroxyle, alcoxy en C₁-C₄.

De préférence, le composé de formule (l) est l'hydroxyde de tétrabutyl ammonium.

Le cas échéant, les composés de formule (l) peuvent être générés in situ dans la composition selon l'invention.

Les hydroxydes de phosphonium quaternaires correspondent à la formule (l') suivante : 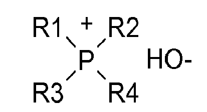 dans laquelle R₁, R₂, R₃, R₄ identiques ou différents représentent des radicaux alkyles en C₁-C₁₀, éventuellement substitués par un ou plusieurs atomes d'halogène, en particulier le chlore, le brome ou l'iode, un ou plusieurs radicaux hydroxyle, alcoxy en C₁-C₄, carboxylique.

De préférence, les radicaux alkyles de la formule (l') sont en C₂-C₆, lesdits radicaux étant de manière encore plus préférée, non substitués.

Selon une variante particulièrement avantageuse, le composé de formule (l') est l'hydroxyde de tétrabutyl phosphonium.

Il est précisé que le composé de formule (l') peut être introduit dans la composition en tant que tel ou bien être préparé in-situ à partir d'un halogénure de phosphonium quaternaire ajouté à la composition selon l'invention, en conditions alcalines.

Selon un mode de réalisation de l'invention, la teneur en composé(s) de formule (l) et/ou (l') représente de 0,01 à 30 % en poids, par rapport au poids de la composition, et de façon encore plus particulière de 0,05 à 15 % en poids par rapport au poids de la composition.

Selon un mode de réalisation de l'invention, la composition comprend en outre un ou plusieurs composés organiques présentant une valeur du paramètre de solubilité δH de Hansen comprise entre 5 et 16 Mpa^{1/2} et de poids moléculaire inférieur à 250 g/mol.

De préférence, lesdits composés organiques présentent une valeur du paramètre de solubilité δH de Hansen comprise entre 5 et 13 de préférence entre 7 et 13 Mpa^{1/2}.

Ces composés organiques sont avantageusement choisis parmi les alcanols aliphatiques, les éthers aliphatiques de mono- ou poly-alcools aliphatiques ou aromatiques, les alcools aromatiques ou les alcools alkylaryle, les carbonates d'alkylène ; ou encore leurs mélanges.

De préférence, ces composés organiques sont choisis parmi le 1-octanol, le 1-décanol, le tridécyl alcool, le dipropylène glycol monométhyléther acétate, le dipropylène glycol méthyléther, le tripropylène glycol méthyléther, le propylène glycol n-butyl éther, le propylène glycol n-propyl éther, le monométhyléther de propylèneglycol, le monoéthyléther et le monométhyléther du diéthylèneglycol, le 3-phényl-1-propanol, le 2-phényl-1-propanol, l'alcool benzylique, le benzyloxyéthanol, le phénoxyéthanol, le carbonate de propylène, ou leurs mélanges.

De préférence, le composé organique, s'il est présent dans la composition selon l'invention, est l'alcool benzylique.

Lorsqu'ils sont présents, la teneur du ou des composés organiques précités varie entre 0,1 et 30 % en poids par rapport au poids de la composition, de préférence entre 0,5 et 20 % en poids par rapport au poids de la composition.

La composition tinctoriale selon l'invention peut également comprendre un ou plusieurs agents de conditionnement.

A titre d'exemple, on peut citer les silicones linéaires, cyclique, ramifiées ou non ramifiées, volatiles ou non volatiles. Ces silicones peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C.,

A titre d'agent de conditionnement, on peut aussi utiliser les polymères cationiques tels que les polyquaterniums 22, 6, 10, 11, 35 et 37 et le chlorure d'hexadimethrine.

La concentration en agent(s) de conditionnement dans la ou les compositions utiles dans l'invention peut varier de 0,01 à 10% en poids par rapport au poids total de la composition, de préférence de 0,05 à 5% et plus préférentiellement encore de 0,1 à 3%.

Les compositions selon l'invention peuvent contenir en outre un ou plusieurs agents épaississants organiques.

Les agents épaississants organiques peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères amphiphiles comprenant des zones hydrophiles, et des zones hydrophobes à chaîne grasse capables, dans un milieu aqueux, de s'associer réversiblement entre eux ou avec d'autres molécules).

Selon un mode de réalisation particulier, l'épaississant est polymérique et choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique.

En ce qui concerne les agents épaississants associatifs, on peut mettre en oeuvre un ou plusieurs polymères de nature non ionique ou ionique, de préférence anionique ou cationique.

Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères associatifs anioniques comportant au moins une chaîne grasse, on peut citer :
- (l) les polymères comportant au moins un motif hydrophile, et au moins un motif éther d'allyle à chaîne grasse, plus particulièrement ceux dont le motif hydrophile est constitué par un monomère anionique insaturé éthylénique, avantageusement par un acide carboxylique vinylique et tout particulièrement par un acide acrylique ou un acide méthacrylique ou les mélanges de ceux ci, et dont le motif éther d'allyle à chaîne grasse correspond au monomère de formule (A) suivante :

   CH₂ = C R' CH₂ O Bₙ R (A)

   dans laquelle R' désigne H ou CH₃, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (A) plus particulièrement préféré est un motif dans lequel R' désigne H, n est égal à 10, et R désigne un radical stéaryl (C₁₈).
   Parmi ces polymères anioniques à chaîne grasse, on préfère les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (A), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
- (ll) les polymères comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (B) suivante : 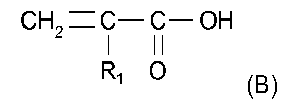 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (C) suivante : 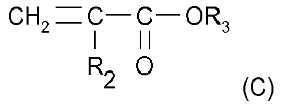 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Les esters d'alkyles (C₁₀-C₃₀) d'acides carboxyliques insaturés sont par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Parmi ce type de polymères anioniques à chaîne grasse, on utilisera plus particulièrement des polymères formés à partir d'un mélange de monomères comprenant :
   (i) essentiellement de l'acide acrylique,
   (ii) un ester de formule (C) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ce type de polymères anioniques à chaîne grasse, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.
   Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX .
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.
- (IV) les terpolymères acryliques comprenant :
   (a) 20% à 70% en poids d'un acide carboxylique à insaturation α,β-monoéthylénique,
   (b) 20 à 80% en poids d'un monomère à insaturation α,β-monoéthylénique non-tensio-actif différent de (a),
   (c) 0,5 à 60% en poids d'un mono-uréthane non-ionique qui est le produit de réaction d'un tensio-actif monohydrique avec un monoisocyanate à insaturation monoéthylénique,
      tels que ceux décrits dans la demande de brevet EP-A-0173109 et plus particulièrement un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras (C₈-C₃₀)oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères associatifs non ioniques comportant au moins une chaîne grasse sont choisis de préférence parmi :
- (1) les celluloses modifiées par des groupements comportant au moins une chaîne grasse, comme notamment ;
- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
- celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
- (3) les copolymères de vinyl pyrrolidone et de monomères hydrophobes à chaîne grasse,
   avec par exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polymères à squelette aminoplaste éther possédant au moins une chaîne grasse, tels que les composés PURE THIX proposés par la société SUD-CHEMIE.
- (7) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes grasses hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

A titre d'exemples de polyéthers polyuréthanes non ioniques à chaîne grasse utilisables dans l'invention, on peut utiliser le Rhéolate 205 à fonction urée vendu par la société RHEOX ou encore les Rhéolates 208 , 204 ou 212, ainsi que l'Acrysol RM 184, l'Aculyn ou Acrysol 44 et l'Aculyn ou Acrysol 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en C₁₂-C ₁₄ et le produit ELFACOS T212 à chaîne alkyle en C₁₈ de chez AKZO, ainsi que le produit DW 1206B de chez ROHM & HAAS à chaîne alkyle en C₂₀ et à liaison uréthanne, proposé à 20 % en matière sèche dans l'eau.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères associatifs cationiques comportant au moins une chaîne grasse utilisés peuvent notamment être choisis parmi les dérivés de cellulose quaternisée, les polyuréthanes cationiques, les polyvinyllactames cationiques, et de préférence, parmi les dérivés de cellulose quaternisée.

A tire d'exemple de polymères de ce type, on peut citer en particulier :
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

La teneur en polymères épaississants, s'ils sont présents, varie habituellement de 0,05 % à 5 % en poids, par rapport au poids de la composition de coloration.

Selon un mode de réalisation particulièrement avantageux, la composition tinctoriale comprend un ou plusieurs agents tensioactifs. Ces derniers peuvent être indifféremment choisis, seuls ou en mélanges, parmi des tensioactifs anioniques, amphotères, non ioniques, zwittérioniques et cationiques.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :
- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamide-sulfosuccinates ;
- les alkylsulfoacétates ;
- les acylsarconisates ; et les acylglutamates :
- les esters d'alkyle et d'acides polyglycoside-carboxyliques tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les acides d'alkyl-D-galactoside uroniques ;
- les acides alkyléther-carboxyliques polyoxyalkylénés, les acides alkylaryléther-carboxyliques polyoxyalkylénés, les acides alkylamidoéther carboxyliques polyoxyalkylénés ;
   le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :
- les alcools gras polyéthoxylés, polypropoxylés ou polyglycérolés,
- les alpha-diols polyéthoxylés, polypropoxylés ou polyglycérolés,
   le nombre de groupements oxyde d'éthylène ou oxyde de propylène allant de 2 à 50 ; le nombre de groupements glycérol allant de 2 à 30 ;
- les condensats d'oxyde d'éthylène et d'oxyde de propylène sur des alcools gras ;
- les amides gras polyéthoxylés ayant de 2 à 30 moles d'oxyde d'éthylène ;
- les amides gras polyglycérolés comportant de 1 à 5 groupements glycérol ;
- les esters éthoxylés d'acides gras du sorbitane ayant de 2 à 30 moles d'oxyde d'éthylène, les esters d'acides gras du saccharose ;
- les alkylpolyglucosides, les dérivés de N-alkylglucamine ;
   ces composés comprenant au moins une chaîne alkyle ou alcényle comprenant 10 à 24 atomes de carbone.
- les copolymères d'oxyde d'éthylène et d'oxyde de propylène.

Les tensioactifs cationiques entrant dans la composition selon l'invention, peuvent notamment être choisis parmi les composés suivants, seuls ou en mélange :
- les amines grasses primaires, secondaires ou tertiaires éventuellement polyéthoxylées (2 à 30 moles d'oxyde d'éthylène) et leurs sels
- les sels d'ammonium quaternaire comprenant ou non une ou plusieurs fonctions ester tels que les chlorures ou les bromures de tétraalkylammonium, d'alkylamidoalkyltrialkylammonium, de trialkylbenzyl-ammonium, de trialkylhydroxyalkylammonium, d'alkylpyridinium, et les diesters quaternaires.
- les dérivés d'alkyl-imidazoline ;
   ces composés comprenant au moins une chaîne alkyle comprenant 10 à 24 atomes de carbone.

Enfin, les tensioactifs amphotères peuvent être choisis parmi les composés suivants, seuls ou en mélange :
- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les aikyiamidoaikyi(C6-C8)bétaïnes, les alkylamidoalkyl(C₆-C₈)sulfobétaïnes ;
   ces composés comprenant une au moins chaîne alkyle comprenant de 10 à 24 atomes de carbone.

De préférence, les tensioactifs sont non ioniques, anioniques ou amphotères et de manière encore plus préférée, non ioniques.

Habituellement, les agents tensioactifs représentent une quantité comprise entre 0,01 et 50 % en poids, de préférence entre 0,1 et 25 % en poids par rapport au poids de la composition.

La composition tinctoriale conforme à l'invention peut également renfermer divers adjuvants utilisés classiquement dans les compositions pour la teinture des cheveux, tels que par exemple des polymères anioniques, non ioniques, amphotères, zwitterioniques différents des épaississants mentionnés auparavant, ou leurs mélanges ; des agents épaississants minéraux comme notamment les argiles ; des agents antioxydants tels que par exemple l'acide ascorbique, l'acide érythorbique ; des agents réducteurs avec entre autres le sulfite, bisulfite ou métabisulfite d'ammonium, le thiolactate d'ammonium ; des agents de pénétration, des agents séquestrants comme l'éthylènediamine tétraacétique ou ses sels ; des parfums ; des agents matifiants avec par exemple les oxydes de titane ; des tampons ; des agents dispersants ; des agents filmogènes ; des céramides ; des acides carboxyliques notamment aromatiques comme l'acide benzoïque ; et des agents conservateurs.

Les adjuvants ci dessus sont en général présents en quantité comprise pour chacun d'eux entre 0,01 et 20 % en poids par rapport au poids de la composition.

Le milieu cosmétiquement acceptable de la composition, qui est un milieu approprié pour la coloration des fibres kératiniques humaines, comprend de préférence de l'eau et éventuellement un ou plusieurs solvants additionnels incluant les composés organiques précités avec paramètre de Hansen particulier.

A titre d'exemples de tels solvants additionnels autres que ces composés organiques particuliers, on peut par exemple citer, les alcanols, linéaires ou ramifiés, en C₂-C₄, tels que l'éthanol et l'isopropanol ; les polyols et éthers de polyols comme le 2-butoxyéthanol l'hexylène glycol, le propylèneglycol, le dipropylèneglycol, le glycérol, et leurs mélanges.

Le ou les solvants additionnels peuvent être présents dans des proportions allant de préférence de 0,1 à 30 % en poids par rapport au poids total de la composition tinctoriale, et encore plus préférentiellement de 0.5 à 20 % en poids.

Il est à noter que la quantité d'eau dans la composition selon l'invention est de préférence supérieure à 10 % en poids par rapport au poids de la composition, et plus avantageusement supérieure ou égale à 25% en poids. De préférence, la teneur en eau est comprise entre 25 et 98 % en poids par rapport au poids de la composition.

Le pH de la composition selon l'invention peut être compris entre 2 et 13. Il est plus préférentiellement compris entre 5 et 11.

Il peut être ajusté à la valeur désirée au moyen d'un ou plusieurs agents acidifiants ou d'un ou plusieurs agents alcalinisants habituellement utilisés dans le domaine.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, outre le composé de formule (l), l'ammoniaque, les carbonates alcalins, les alcanolamines telles que les mono-, di- et triéthanolamines ainsi que leurs dérivés, les hydroxydes de sodium ou de potassium et les composés de formule suivante : 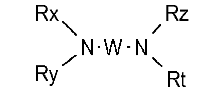 dans laquelle W est un reste propylène éventuellement substitué par un groupement hydroxyle ou un radical alkyle en C₁-C₆ Rx, Ry, Rz et Rt, identiques ou différents, représentent un atome d'hydrogène, un radical alkyle en C₁-C₆ ou hydroxyalkyle en C₁-C₆.

Si la composition comprend de l'ammoniaque comme agent alcalinisant, sa teneur pondérale (exprimée en NH₃) ou l'un de ses sels, reste de préférence inférieure à la teneur pondérale de composé(s) de formule (l) et inférieure à la teneur pondérale totale d'agent(s) alcalinisant(s), le ou les composés de formule (l) étant compris.

La composition selon l'invention peut comprendre également un ou plusieurs agents oxydants. On parle dans ce cas de composition prête à l'emploi.

En particulier, la composition prête à l'emploi est obtenue par mélange extemporané avant l'application, d'une composition précédemment décrite, avec au moins une composition comprenant, dans un milieu cosmétiquement acceptable, un ou plusieurs agents oxydants.

L'agent oxydant est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium.

L'utilisation du peroxyde d'hydrogène est particulièrement préférée.

Cet agent oxydant est avantageusement constitué du peroxyde d'hydrogène en solution aqueuse (eau oxygénée) dont le titre peut varier, plus particulièrement, de 1 à 40 volumes, et encore plus préférentiellement de 5 à 40 volumes.

Les compostions selon l'invention peuvent résulter du mélange extemporané de plusieurs compositions.

Un autre objet de l'invention est donc constitué par un procédé de coloration de fibres kératiniques qui consiste à appliquer la composition décrite précédemment.

Conformément à un premier mode de réalisation, la composition appliquée ne comprend pas d'agent oxydant. Cette variante est appropriée lorsque la composition ne comprend pas de précurseurs de colorants d'oxydation, qui sont des composés choisis parmi les bases d'oxydation et les coupleurs.

A titre d'exemples de bases d'oxydation classiquement employées, on peut citer les paraphénylènediamines, les bis-phénylalkylènediamines, les para-aminophénols, les ortho-aminophénols, les bases hétérocycliques et leurs sels d'addition.

A titre de coupleurs, on peut notamment citer les méta-phénylènediamines, les méta-aminophénols, les méta-diphénols, les coupleurs naphtaléniques, les coupleurs hétérocycliques ainsi que leurs sels d'addition.

Conformément à un deuxième mode de réalisation de l'invention, la composition est appliquée en présence d'au moins un agent oxydant. Ce mode de réalisation est approprié pour tout type de colorant capillaire.

Selon une première variante de ce deuxième mode, on applique sur les fibres la composition prête à l'emploi qui vient d'être détaillée et qui est obtenue par mélange extemporané avant l'application, d'une composition selon l'invention dépourvue d'agent oxydant avec une composition oxydante.

Selon une deuxième variante, on applique la composition selon l'invention dépourvue d'agent oxydant, et une composition oxydante, successivement et sans rinçage intermédiaire.

La composition oxydante mise en oeuvre comprend un ou plusieurs agents oxydants tels que définis plus haut.

Concernant les solvants organiques éventuellement présents dans la composition oxydante, on pourra se reporter à la liste indiquée auparavant dans le cadre du descriptif de la composition selon l'invention.

Habituellement, le pH de la composition oxydante est inférieur à 7.

La composition oxydante peut se présenter sous la forme d'une solution, une émulsion ou un gel.

Elle peut éventuellement comprendre un ou plusieurs additifs utilisés classiquement dans le domaine de la coloration des fibres kératiniques humaines, en fonction de la forme galénique souhaitée. On pourra là encore se reporter à la liste des additifs donnée plus haut.

Le mélange appliqué sur les fibres est laissé en place pour une durée, en général, de l'ordre de 1 minute à 1 heure, de préférence de 10 minutes à 30 minutes.

La température durant le procédé est classiquement comprise entre 10 et 200°C et plus particulièrement comprise entre la température ambiante (entre 15 à 25°C) et 80°C, de préférence entre la température ambiante et 60°C.

A l'issue du traitement, les fibres kératiniques humaines sont éventuellement rincées à l'eau, lavées au shampooing, rincées à nouveau à l'eau puis séchées ou laissées à sécher.

De préférence les fibres kératiniques humaines sont les cheveux.

Des exemples concrets mais non limitatifs de l'invention vont maintenant être présentés.

### EXEMPLES

### Exemple 1 :

On prépare les compositions de coloration suivantes à partir des ingrédients dans les proportions indiquées:

| | **A(comparatif)** | **B(invention)** |
|---|---|---|
| Para-phénylènediamine | 0,002mol | 0,002mol |
| Dichlorhydrate de 2,4 diamino phénoxyéthanol | 0,002mol | 0,002mol |
| Alcool benzylique | 5g | 5g |
| Hydroxyde de tétrabutylammonium | - | 0,013 mol |
| Ammoniac | 0,013 mol | - |
| Eau | qsp 100g | qsp 100g |

On mélange chacune des compositions A à B poids pour poids avec de l'eau oxygénée à 20 volumes à pH=2,2.

On applique ensuite chacun des mélanges sur des mèches de cheveux à 90% de cheveux blancs naturels et permanentés pendant 30 minutes à température ambiante.

Les mèches sont ensuite essorées, rincées, shampouinées et séchées.

On voit que les mèches colorées par la composition B de l'invention sont significativement plus chromatiques que celles colorées par la coloration comparative A contenant de l'ammoniac comme agent alcalin.

Ceci est confirmé par les mesures colorimétriques (colorimètre Minolta CM2022).

| | **C* sur cheveux naturels** | **C* sur cheveux permanentés** |
|---|---|---|
| **Cheveu coloré par A** | 4,2 | 1,4 |
| **Cheveu coloré par B** | 6,8 | 3,4 |

### Exemple 2 :

On prépare les compositions de coloration suivantes à partir des ingrédients dans les proportions indiquées :

| | **C** | **D** | **E** |
|---|---|---|---|
| Para-phenylènediamine | 0,002 mol | 0,002mol | - |
| Para-aminophénol | - | - | 0,002mol |
| 2-méthyl-5-amino phénol | 0,002mol | - | 0,002 mol |
| Dichlorhydrate de 2,4 diamino phénoxyéthanol | - | 0,002mol | - |
| Alcool benzylique | 5g | 5g | 5g |
| Hydroxyde de tétrabutylammonium | 0,013mol | 0,013mol | 0,013mol |
| Eau | qsp 100g | qsp 100g | qsp 100g |

On mélange chacune des compositions C à E poids pour poids avec de l'eau oxygénée à 20 volumes à pH=2,2.

On applique ensuite chacun des mélanges sur des mèches de cheveux à 90% de cheveux blancs naturels et permanentés pendant 30 minutes à température ambiante.

Les mèches sont ensuite essorées, rincées, shampouinées et séchées.

On obtient à partir des compositions C, D et E respectivement des colorations des mèches rouges violacé (composition C), bleues (composition D), et cuivré (composition E) puissantes et peu sélectives.

### Exemple 3 :

On prépare les compositions de coloration suivantes à partir des ingrédients dans les proportions indiquées :

| | **A1** | **B1** | **C1** | **D1** |
|---|---|---|---|---|
| Colorant 1 (*) | 0,00013 mol | | | |
| Alcool benzylique | 5g | | | |
| Hydroxyde de tétraéthyl ammonium | 0,0013 mol | - | - | - |
| Hydroxyde de tétrapropyl ammonium | - | 0,0013 mol | - | - |
| Hydroxyde de tétrabutyl ammonium | - | - | 0,0013 mol | - |
| Hydroxyde de tétrapentyl ammonium | - | - | - | 0,0013 mol |
| Eau | qsp 100g | qsp 100g | qsp 100g | qsp 100g |

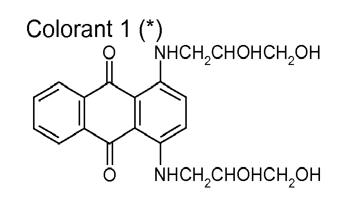

Des mèches de cheveux à 90% de cheveux blancs permanentés sont alors colorées 30 minutes à température ambiante à partir de chacune de ces compositions.

Les mèches sont ensuite essorées, rincées, shampouinées et séchées.

On obtient des colorations des mèches bleues puissantes comme l'indiquent les mesures colorimétriques.

| | **L*** | **a*** | **b*** | **DE / cheveu non coloré** |
|---|---|---|---|---|
| **Cheveu non coloré** | 60,8 | 0,6 | 11,7 | **-** |
| **Cheveu coloré par A1** | 32,3 | -8,6 | -23,7 | **46,4** |
| **Cheveu coloré par B1** | 24,2 | -2,2 | -22,4 | **50,1** |
| **Cheveu coloré par C1** | 26,2 | -2,3 | -25,8 | **51,7** |
| **Cheveu coloré par D1** | 34,6 | -8,2 | -22,0 | **43,6** |

### Exemple 4 :

On prépare les compositions de coloration suivantes à partir des ingrédients dans les proportions indiquées :

| | **E1** | **F1** | **G1** | **H1** |
|---|---|---|---|---|
| Disperse red 13 | 0,00013 mol | - | - | - |
| Disperse red 17 | - | 0,00013 mol | - | - |
| Curcumine | - | - | 0,00013 mol | - |
| Basic red 51 | - | - | - | 0,00013 mol |
| Alcool benzylique | 5g | 5g | 5g | 5g |
| Hydroxyde de tétrabutyl ammonium | 0,0013 mol | 0,0013 mol | 0,0013 mol | 0,0013 mol |
| Eau | qsp 100g | qsp 100g | qsp 100g | qsp 100g |

Des mèches de cheveux à 90% de cheveux blancs naturels sont alors colorées 30 minutes à température ambiante à partir de chacune de ces compositions.

Les mèches sont ensuite essorées, rincées, shampouinées et séchées.

On obtient des colorations des mèches puissantes comme l'indiquent les mesures colorimétriques du tableau ci-dessous.

| | **L*** | **a*** | **b*** | **DE / cheveu non coloré** |
|---|---|---|---|---|
| **Cheveu non coloré** | 61,26 | 0,84 | 13,71 | **-** |
| **Cheveu coloré par E1** | 31,77 | 19,97 | -3,09 | **38,96** |
| **Cheveu coloré par F1** | 23,15 | 20,53 | 3,01 | **44,21** |
| **Cheveu coloré par G1** | 35,61 | 18,11 | 22,73 | **32,21** |
| **Cheveu coloré par H1** | 24,23 | 31,9 | 9,8 | **48,49** |

### Exemple 5 :

On prépare la composition de coloration suivante à partir des ingrédients dans les proportions indiquées :

| | **I1** |
|---|---|
| Colorant direct 1 | 0,00013 mol |
| Alcool benzylique | 5g |
| Hydroxyde de tétrabutyl ammonium | 0,0013 mol |
| Eau | qsp 100g |

Des mèches de cheveux à 90% de cheveux blancs naturels, sont alors colorées 30 minutes à température ambiante en utilisant ladite composition.

Les mèches sont ensuite essorées, rincées, shampouinées et séchées.

Une épreuve de 5 puis 10 shampooings est alors réalisée.

On obtient des colorations des mèches puissantes et tenaces comme l'indiquent les mesures colorimétriques.

| | **DE** | **DE** | **DE** |
|---|---|---|---|
| | avant épreuve | Après épreuve 5 shampooings | Après épreuve 10 shampooings |
| **Cheveu coloré par I1** | 44,71 | 38,26 | 36,07 |

### Exemple 6 :

On prépare les compositions de coloration suivantes à partir des ingrédients dans les proportions indiquées en %:

| | |
|---|---|
| Colorant (*) | 0,0013 mol |
| Alcool benzylique | 5g |
| Bromure de tétrabutylphosphonium | 0,013 mol |
| Soude (1N) | 0.013 mol |
| Eau | qsp 100g |

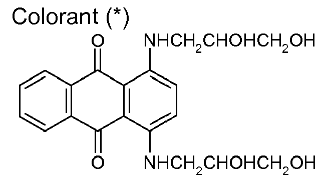

Des mèches de cheveux à 90% de cheveux blancs permanentés sont alors colorées 30 minutes à température ambiante.

Les mèches sont ensuite essorées, rincées, shampouinées et séchées.

On obtient des colorations des mèches bleues puissantes comme l'indiquent les mesures colorimétriques (colorimètre Minolta CM2022)

| | **L*** | **a*** | **b*** | **DE /cheveu non coloré** |
|---|---|---|---|---|
| **Cheveux non colorés** | 60.83 | 0.61 | 11.71 | **-** |
| **Cheveux colorés par la composition** | 25.75 | -3.27 | -24.41 | **50.5** |

## Revendications

1. Composition tinctoriale comprenant un milieu cosmétiquement acceptable, un ou plusieurs colorants capillaires, et un ou plusieurs composés choisis parmi les composés de formules (l) et (l') suivantes
(l) hydroxydes d'ammonium quaternaires de formule (l) suivante : 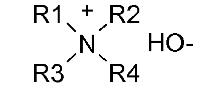 dans laquelle R₁, R₂, R₃, R₄, identiques ou différents représentent des radicaux alkyles en C₁-C₂₀, plus particulièrement en C₁-C₁0 éventuellement substitués par un groupement choisi parmi les atomes d'halogène, les groupements hydroxyle, alcoxy en C₁-C₄ ; des radicaux alkyl(C₁-C₂)aryle (C₆) ;
(l') hydroxyde de phosphonium quaternaire de formule (l') suivante : 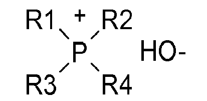 dans laquelle R₁, R₂, R₃, R₄ identiques ou différents représentent des radicaux alkyles en C₁-C₁₀ éventuellement substitués par un ou plusieurs atomes d'halogène, en particulier le chlore, le brome ou l'iode, un ou plusieurs radicaux hydroxyle, alcoxy en C₁-C₄, carboxylique.

2. Composition selon la revendication précédente, **caractérisée en ce que** le ou les colorants d'oxydation sont choisis parmi les bases d'oxydation, les coupleurs, et leurs mélanges.

3. Composition selon la revendication précédente, **caractérisée en ce que** le ou les bases d'oxydation choisies parmi les paraphénylènediamines, les bis-phénylalkylènediamines, les para-aminophénols, les ortho-aminophénols, les bases hétérocycliques et leurs sels d'addition.

4. composition selon la revendication 2, **caractérisée en ce que** les coupleurs sont choisis parmi les méta-phénylènediamines, les méta-aminophénols, les méta-diphénols, les coupleurs naphtaléniques, les coupleurs hétérocycliques ainsi que leurs sels d'addition.

5. Composition selon l'une quelconque des revendications précédentes, **caractérisée en ce que** le ou les colorants directs sont choisis parmi les colorants directs synthétiques, les colorants naturels, non ioniques ou ioniques, ou leurs mélanges.

6. Composition selon la revendication précédente, **caractérisée en ce que** le ou les colorants directs synthétiques sont choisis parmi les colorants nitrés de la série benzénique ; les colorants directs azoïques ; méthiniques ; azométhiniques avec plus particulièrement les diazacarbocyanines et leurs isomères et les tétraazacarbocyanines ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines et porphyrines ; seuls ou en mélanges ; et de préférence parmi les colorants directs cationiques monochromophoriques de types azoïques ; méthines ; azométhines avec les diazacarbocyanines et leurs isomères, les tétraazacarbocyanines ; anthraquinoniques ; seuls ou en mélange.

7. Composition selon l'une quelconque des revendications 5 ou 6, **caractérisée en ce que** le ou les colorants directs sont choisis parmi les composés de formules (A1) à (A6) et les colorants non ioniques de log P supérieur ou égal à 2 : 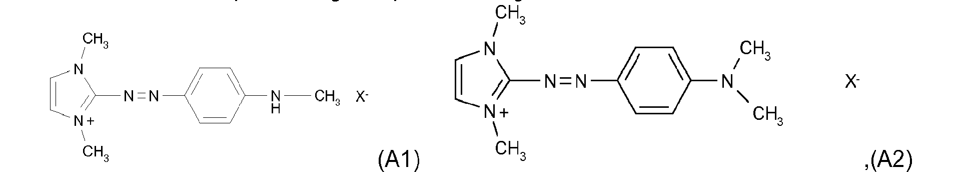 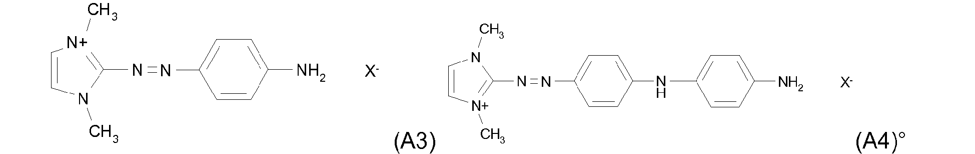 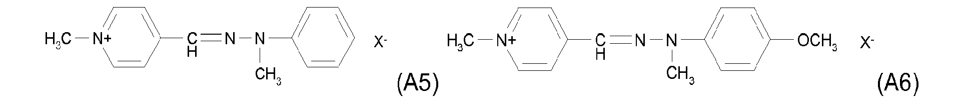 X- représentant un anion ou mélange d'anions cosmétiquement acceptables.

8. Composition selon l'une quelconque des revendications 5 à 7, **caractérisée en ce que** le ou les colorants directs synthétiques sont choisis parmi les colorants non ioniques présentant un LogP supérieur ou égal à 2.

9. Composition selon la revendication 5, **caractérisée en ce que** le ou les colorants directs sont choisis parmi les naturels choisis parmi la lawsone, la juglone, l'alizarine, la purpurine, l'acide carminique, l'acide kermésique, l'acide laccaïque, la purpurogalline, l'anthragallol, la protocatéchaldéhyde, l'indigo, l'isatine, la curcumine, la spinusoline, les chlorophylles, les chlorophyllines, les orcéines, l'hématéine, l'hématoxyline, la braziline, la braziléine, les colorants du carthame, les flavonoïdes, les anthocyanes, les caroténoïdes, les tanins, les extraits de sorgho et le carmin de cochenille, ou leurs mélanges.

10. Composition selon l'une quelconque des revendications précédentes, **caractérisée en ce que** le ou les composés de formule (l) sont choisis .parmi l'hydroxyde de méthyltriéthanol ammonium, l'hydroxyde de tétraéthyl ammonium, l'hydroxyde de benzyltriméthyl ammonium, l'hydroxyde de tétraméthyl ammonium, l'hydroxyde de tétrabutyl ammonium, l'hydroxyde de tétrapropyl ammonium, l'hydroxyde de méthyl triéthanol ammonium, l'hydroxyde de benzyl diméthyl éthanol ammonium, l'hydroxyde de benzyl triéthyl ammonium, l'hydroxyde de benzyl tributyl ammonium, l'hydroxyde d'octadécyl triméthyl ammonium, l'hydroxyde de tétrahexyl ammonium, l'hydroxyde de tétraoctyl ammonium, ou leurs mélanges.

11. Composition selon l'une quelconque des revendications précédentes, **caractérisée en ce que** le ou les composés de formule (l) sont tels que les radicaux R₁, R₂, R₃, R₄, identiques ou différents représentent des radicaux alkyles en C₂-C₆, éventuellement substitués par un groupement choisi parmi les groupements hydroxyle, alcoxy en C₁-C₄.

12. Composition selon l'une quelconque des revendications précédentes, **caractérisée en ce que** la teneur en composé(s) de formules (l) et (l') représente 0,01 à 30 % en poids, par rapport au poids de la composition, et de préférence de 0,05 à 15 % en poids par rapport au poids de la composition.

13. Composition selon l'une quelconque des revendications précédentes, **caractérisée en ce qu'**elle comprend un ou plusieurs composés organiques présentant une valeur du paramètre de solubilité δH de Hansen comprise entre 5 et 16 Mpa^{1/2}, de préférence comprise entre 5 et 13, plus particulièrement comprise entre 7 et 13 Mpa^{1/2}, et de poids moléculaire inférieur à 250 g/mol.

14. Composition selon la revendication 13, **caractérisée en ce que** le ou les composés organiques sont choisis parmi les alcanols aliphatiques, les éthers aliphatiques de mono-ou poly-alcools aliphatiques ou aromatiques, les alcools aromatiques ou les alcools alkylaryle, les carbonate d'alkylène ; ou encore leurs mélanges.

15. Composition selon l'une quelconque des revendications 13 ou 14, **caractérisée en ce que** les composés organiques (II) sont choisis parmi le 1-octanol, le 1-décanol, le tridécyl alcool, le dipropylène glycol monométhyléther acétate, le dipropylène glycol méthyléther, le tripropylène glycol méthyléther, le propylène glycol n-butyl éther, le propylène glycol n-propyl éther, le monométhyléther de propylèneglycol, le monoéthyléther et le monométhyléther du diéthylèneglycol, le 3-phényl-1-propanol, le 2-phényl-1-propanol, l'alcool benzylique, le benzyloxyéthanol, le phénoxyéthanol, le carbonate de propylène, ou leurs mélanges.

16. Composition selon l'une quelconque des revendications 13 à 15, **caractérisée en ce que** la teneur du ou des composés organiques varie 0,1 et 30% en poids par rapport au poids de la composition, de préférence entre 0,5 et 20 % en poids par rapport au poids de la composition.

17. Composition selon l'une quelconque des revendications précédentes, **caractérisée en ce qu'**elle comprend un ou plusieurs agents oxydants.

18. Procédé de coloration consistant à appliquer sur les fibres kératiniques humaines, une composition selon l'une quelconque des revendications précédentes.

19. Dispositif multi-compartiments comprenant au moins une composition selon l'une quelconque des revendications 1 à 16 et une composition oxydante comprenant, dans un milieu cosmétiquement acceptable, un ou plusieurs agents oxydants.
